# EUROPEAN PATENT SPECIFICATION

(11) **EP 0 935 948 B1**
(45) Date of publication and mention of the grant of the patent: **24.09.2003**
(21) Application number: 97917477.8
(22) Date of filing: 11.04.1997
(51) Int. Cl.: A61B 17/17

(54) **SYSTEM FOR THE TREATMENT OF FRACTURES AND OTHER INJURIES OF THE FEMUR, TIBIA AND HUMERUS**
BEHANDLUNGSSYSTEM FÜR FRAKTUREN UND ANDERE VERLETZUNGEN DES FEMURS,DER TIBIA UNDDES HUMERUS
SYSTEME DE TRAITEMENT DE FRACTURES ET D'AUTRES LESIONS DU FEMUR, DU TIBIA ET DE L'HUMERUS

(43) Date of publication of application: 18.08.1999
(73) Proprietor: Colchero Rosas, Fernando, Delegacion Benito Juarez, Mexico, D.F. 03100 (MX)
(72) Inventor: Colchero Rosas, Fernando, Delegacion Benito Juarez, Mexico, D.F. 03100 (MX)
(74) Representative: EGLI-EUROPEAN PATENT ATTORNEYS
(86) International application number: MX9700011
(87) International publication number: WO98046145

## Description

### TECHNICAL FIELD

This invention is focused on the field of medicine, and more specifically, on orthopedic traumatology, because it is a system used to place metal rods with orifices in bones. These rods are secured with bolts that cross the bones and the orifices of the rods. In orthopedics these rods are called nails. The compound of bone, nail and bolts form a single structure that perfectly stabilizes a fracture.

This invention will be used in the treatment of recent fractures ; fractures that did not consolidate ; bone loss caused by infections, tumors or for other reasons ; and asymmetries (a bone longer than its corresponding bone).

### ANTECEDENTS OF THE INVENTION

Systems for treating fractures are disclosed in WO96/03085 and EP-A-0 361 641 which, however, do not provide a series of longitudinal horizontal parallel cavities in a securing body receiving one end of a nail, wherein the levels of the cavities correspond to a scale marking of a bar for holding the other end of the nail.

The nails and bolts are made of stainless steel 316L, which is bio-compatible with the human being and accepted worldwide within the manufacturing standards on implants for orthopedics. Devices that are not left inside the patient's organism are made of another kind of steel, as for example 304.

So far in orthopedics, in the treatment of some injuries in bones, fixed nails with screws at the bone are used, called interlocking nails. These bones have orifices for the screws to enter. The use of these nails requires locating the orifices that they include in order to make the screws enter them when the nail has already been introduced in the marrow channel of the bone that has the injury to be treated. At the present time the location is made using X-ray equipment with a TV monitor, a monitor that is normally called "image intensifier", where one can see the bone and the nail with their orifices.

At the present time there are two kinds of interlocking nails : 1) Hollow nails that require expanding the marrow channel in order to introduce them in the bone, 2) solid nails that penetrate the marrow channel without drilling.

### DESCRIPTION OF THE INVENTION

The invention provides a system as defined in claim 1.

The method followed to locate nails already inserted in the marrow described above presents the problem that said location has a high frequency of failures, above all, because of the mistake in parallelism. In addition to requiring an X-ray or using the image intensifier one or more times in order to provide the distance that exists from the outside part of the bone to the center of the orifice that will be searched and with this measurement, another instrument, that already exists in the state of the technique, places a perforating guide over the orifice and it is perforation with a drill in order to further introduce a bolt. There already exists a family of patents that protects this methodology, formed by Mexican, United States, French, English, Spanish, German and Italian patents, where the holder is the same inventor of the description contained in this application.

Furthermore, when an instrument with orifices (guide) is placed in another instrument for securing purposes and a nail is placed in it, one will logically expect that if the system is properly manufactured, the orifices of the guide and those of the nail will coincide ; however, after many tests we were able to prove that any intra-marrow nail can bend because of the effect of the bone curvature with the result that this coincidence does not occur.

In view of the above, application of this methodology results in the surgeon receiving constant radiation with the corresponding consequences of his health.

In addition, hollow nails are weak because the walls are thin and they break easily when the patient walks. Solid nails without drilling are still weaker because their diameter can be very reduced in order to penetrate the marrow channel without expanding it. Obviously, their weakest areas are located in the orifices of the nail because it has less material at this level and if the orifices are located near the fracture, because of the movements required when walking, the handle arms easily break the nails.

### PURPOSES OF THE INVENTION

The main purpose of this invention is to provide a system for treating fractures and other injuries in bones without requiring an X-ray or image intensifier.

Another purpose of this invention is to give this system features such as the fact that the location of the orifices of the nails inserted in the marrow channel of bones and the closeness of these orifices to the fracture is made in a simple and safe manner.

An additional purpose of this invention is to permit a more resistant system that will assure the integrity of the nails because they are more resistant.

An additional purpose is to give the system the quality of allowing movement of the instrument used to perforate until the orifices coincide with those of the nail, whatever the deformation.

Still an additional purpose is to provide a system that will permit the patient to walk with total support of the injured limb three days after surgery or inclusive, in bone loss.

### BRIEF DESCRIPTION OF THE FIGURES

For a better understanding of the invention we will not describe the figures, in an illustrated but not limited manner.
Figure 1 shows the three kinds of nails that form part of this invention, the higher nail is for injuries in the femur, the middle nail is for injuries of the tibia and the lower one is for use in injuries of the humerus.
Figure 2 shows the aspect of the bolts.
Figure 3 is a conventional view of the vertical component of the securing device of the perforated guide and of the nail of the system that is meant to be protected by this application of patent.
Figure 4 shows two side views of the component illustrated in figure 3.
Figure 5 is the aspect of the horizontal component of the fastener of the perforated guide and of the nail of the system.
Figure 6 illustrates the perforated guide with its different elements.
Figure 7 is a conventional view of the instrument grounds of this system.
Figure 8 represents the guides of drills and bolts.

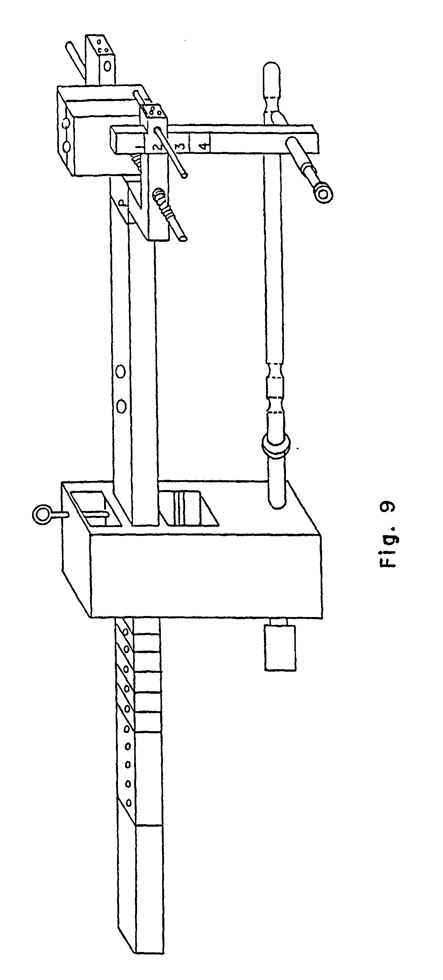

Figure 9 is the complete system placed in a position for surgery.

### DETAILED DESCRIPTION OF THE INVENTION

### Nails of the System

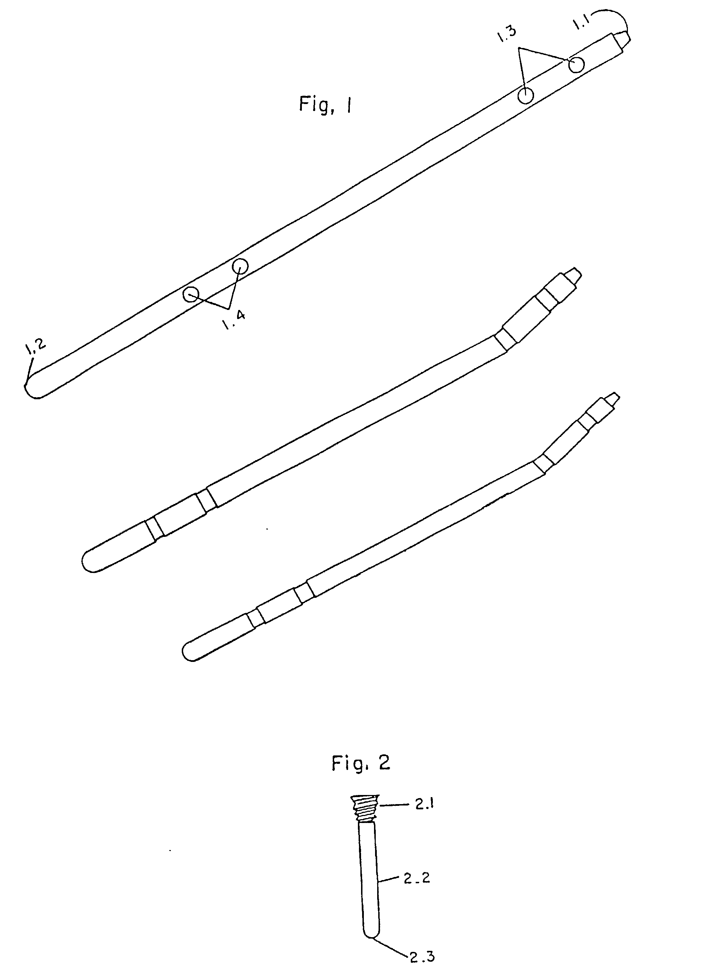

Figure 1 shows the extreme of nail 1.1 (proximal extreme) which is secured with the "nail securing device and the perforated guide" and extreme 1.2 (distal extreme) which has the form of a bullet head or more rounded, so that it will adequately slide inside the marrow channel of the bone. Each nail is perforated in four parts, two parts (orifices 1.3 called proximal orifices), closer to extreme 1.1 and two (orifices 1.4 called distal orifices) closer to extreme 1.2.

These nails are solid rods that require drilling of the bone, of stainless steel 316L, which can have a cylinder shape or another geometric form, straight or curved, with a variable diameter according to the bone where they will be implanted. In the proximal extreme the nail has female threading where the horizontal component of the fastening element of the perforated guide and of the nail will be inserted.

The system includes two types of nails, depending on whether they will be implanted in the femur, tibia or humerus. For each of these types there is a set of nails with different dimensions (the different dimensions will correspond to a different number of nail) so that the surgeon will always have a nail that is adequate for the injury which he will treat.

Although the dimension of the nails may vary according to the bone and the injury that is to be treated, the nails of this system have certain constant characteristics so that they will fulfill their purpose. For example, the distance between each pair of orifices, for any kind and dimension of nail, whether these are distal orifices (1.4) or proximal orifices (1.3), is always the same. For nails of the femur and tibia, the distance between the inside orifices (distal and proximal) is always the same ; and although the distance between these inside orifices smaller in nails for the humerus, this distance is the same for all nails for the humerus. The distance between the outside distal orifices and the distal extreme of the nail is a constant characteristic in any number (dimension) and type (femur, tibia and humerus) of nail. With the type and number of nail, what will vary is the distance between the proximal extreme of the nail and its proximal orifice.

### The bolt of the System

In figure 2 one can appreciate that the bolts are cylindrical rods with a conic extreme (2.1) or conic head, threaded with a configuration that permits it to be turned in order to screw it on to the bone, this may be the configuration required in order to insert an Allen wrench. The bolt also has a smooth body (2.2) with its diameter corresponding to the orifices of the nails. In the opposite extreme to the conic head the bolt ends in a rounded edge. The length of these bolts will vary according to the diameter of the bone where they are to be placed.

### Instruments used to Place and Secure the Nails and the Bolt in the Bone

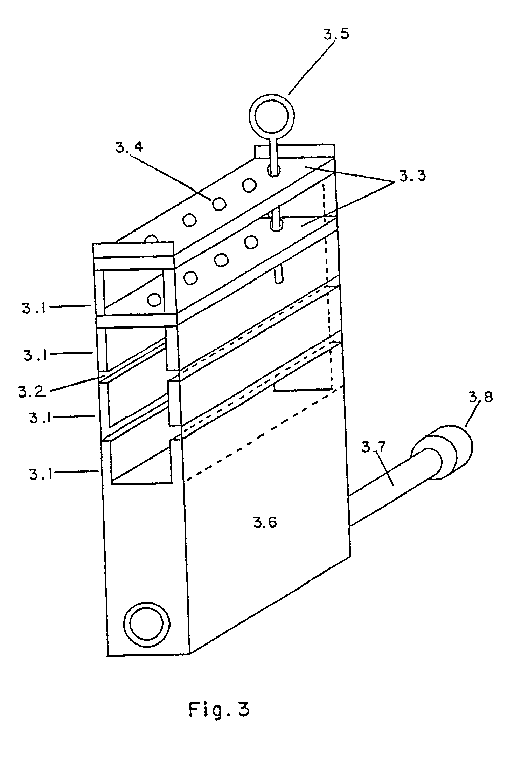

Figures 3 through 8 illustrate the different parts that form the instruments used to place and secure the nails and bolts in the bone. For description purposes these instruments will be divided in three parts : 1) Fastening element of the perforated guide and the nail, 2) the perforated guide and 3) the basic instrument.

### Securing device of the Perforated Guide and of the Nail

Figures 3 to 5 illustrate the different elements that form the "securing device of the perforated guide and of the nail". This instrument provides securing of the "perforated guide" in the front part (referring to the tibia) and side (femur), and in the back part (tibia) and median (femur) to an intra-marrow nail, and has two components : one vertical, which when applied in the operation is placed perpendicular to the bone and another horizontal, that is placed parallel to the bone itself.

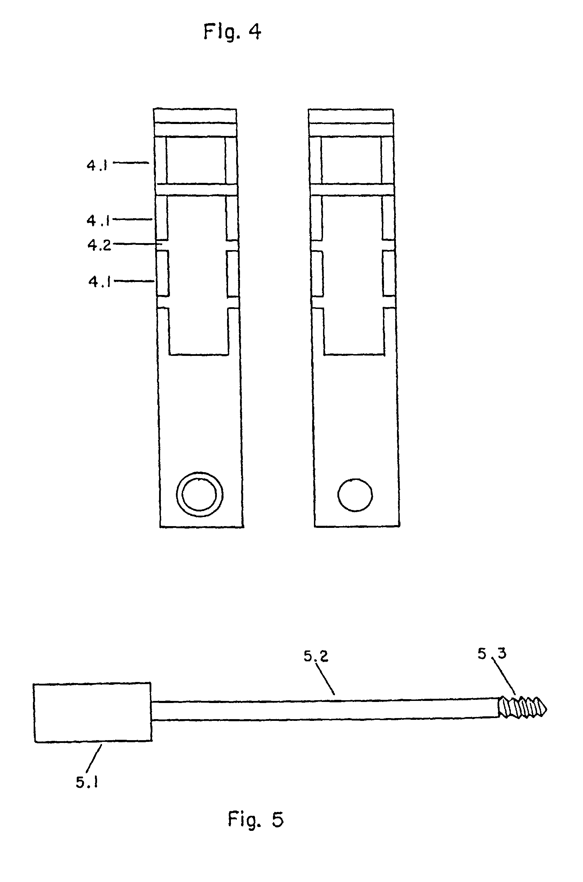

Figures 3 and 4 illustrate the vertical component of the securing device and figure 4 shows the horizontal component ; figure 4 shows the left and right side faces of the vertical component.

In figure 3 one can appreciate that the vertical component adopts the general form of a rectangular or paralelepiped rectangle prism. This instrument is made to provide four different levels (3.1 and 4.1) where to fit in the "perforated guide", bearing a number : level 1, level 2, level 3 and level 4, choosing one where to place the perforated guide. Level 1 is the front (tibia) or side (femur) and level 4 is the back (tibia) or medial (femur). The separation 4.2 between each level with its immediate above and immediate below is approximately 5 mm.

Sheets or soleplates (3.3) are placed in the separations between levels of the perforated guide, with a sheet or soleplate placed above and another below the "perforated guide". Each sheet or soleplate can have five smooth orifices (3.4) with a variable diameter, which will lengthwise have the same distance to the center of same. The distal and proximal orifices are located at a distance of the corresponding extremes of the sheet or soleplate equal or above a tenth of the length of sheet or soleplate.

A bolt (3.5) with a diameter equal to that of the orifices is passed through each of the three distal or proximal orifices.

Starting from where level 4 ends, downwards the vertical component becomes a single part (3.6) and this is the component which, with the orifices that have the sheets or soleplates in the same distribution, provides support to the perforated guide in level 4.

Close to the lower extreme where the vertical component becomes a single part, in the middle part of its width, there is a cavity where a tube is lodged (3.7) with the required inside diameter so that the proximal extreme of the nail will enter its distal extreme, and an outside diameter slightly less than the diameter of the cavity. The length of this 3.7 tube is approximately double the length of the vertical component, with one half of its length jutting out toward the distal, and approximately one tenth of its length toward the proximal when it is inserted in said vertical component. The screw that will be secured to the nail is introduced in the proximal extreme of the tube.

At the level where the nail was secured, in the distal extreme of the tube of the vertical component, said tube has an increase (3.8) in the outside diameter equal to one half of its normal diameter, throughout approximately one tenth of its length, which has the purpose of avoiding that the securing device be introduced in the marrow channel and that the nail is placed overly inside or overly outside. This projecting part is called the bumper.

The function of this "securing device of the perforated guide and of the nails" is to allow the introduction of the perforated guide in the vertical component from the front or side part toward the back or medial part and through the levels it can be placed close to or distant from the patient's skin. If the perforated guide is inserted in level 1, this will be distant from the bone, but if it is inserted in level 4, it will be closer. The closer it can be placed during surgery, the minor space there will be between the "perforated guide" and the bone, and less possibilities of small movements occurring in the instruments.

The horizontal component (figure 5) is formed by a metal cylinder which we can divide into two parts. The proximal part 5.1 is a handle with a larger diameter than the other part, called the distal part, which also has a cylinder shape. The length of the handle is approximately double its diameter. The distal part has the same length as tube 3.7 of the vertical component and has in its extreme opposite to the handle, a threading that is secured in the female threading of the nail.

### Perforated Guide

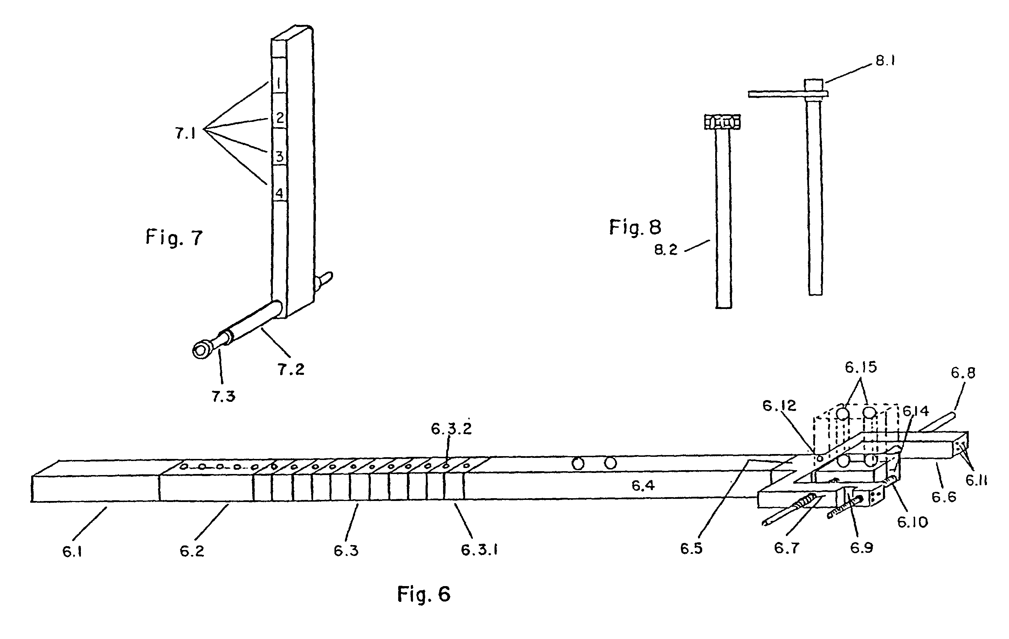

Figure 6 illustrates the perforated guide which, in the case of operations of the femur is longer than in operations of the tibia, and is longer than the one used in operations of the humerus. While different in length, they are the same in all else. The guide is divided into two parts : a long proximal and a short distal. We will name the proximal the proximal body of guide 6.1 and will name the distal perforation body 6.5.

The proximal body has the form of a quadrangular prism where with a length considerably above the length of the nail and the horizontal component of the fastener together. This body has four sections, located in the opposite extreme of the perforation body of section A 6.1, where the function is to provide balance to the guide in order to produce a counterweight. The section next to section A, called section B (6.2), has distributed in the same distances 5 perforations throughout its length through which the bolts will pass which also cross through the sheets or soleplates of the vertical component. This section B will be fastened in the vertical component when nail number 12 (the longest according to our system) is used.

Section C (6.3) of the proximal body of the perforated guide is adjacent toward distal of section B and has a length above the double of section B. This section has a series of numbers engraved that correspond to the number of the nail that is to be inserted in the marrow of the bone. Following this specific guide for each bone (femur, tibia and humerus) each number corresponds to the length of the nail to be used. The numbering is defined by an engraved line transveral to the major axis of the guide accompanied by the number of the nail that corresponds to it. In addition to the number and the engraved line, this section has a series of perforations, one corresponding to each nail number, that also correspond to the perforations of the sheets or soleplates of the vertical component of the securing device.

Section D (6.4) adjacent to the most distal part of the proximal body of the perforated guide has two orifices in its central longitudinal part, with a distance between them equal to the distance that exists between the proximal orifices of the nails of this system. The function of this section of the guide is to locate the proximal orifices of the nail that is being implanted.

Finally, section E (6.5) of the proximal body of the guide has a special mark "P", in order to locate the proximal orifices of the nail when this mark is placed on the distal border of the vertical component. This section is adjacent to section D toward its distal extreme. From the distal edge of the proximal body in its side extremes each have perpendicular arms jutting out in a side direction which we will name measuring rods (6.6) with a height of approximately one half of the rest of the perforated guide. These rods are extended toward each side with a length slightly less than the width of the rest of the body of the perforated guide. The extremes of these arms, at 90° in respect to the arms, in distal direction each, include projections of the same transveral section as the arms, with such a length that they surpass the distal edge of the perforation body.

The right projection, which corresponds to the middle point of the perforation body has a perforation from side to side, perpendicular in horizontal direction, with the same diameter as the perforations of section B and C of the perforation guide, said perforations have a female threading through which a nail (6.7) passes in order to move the perforation body. The threading of the screw and the threading of the perforation of the projection is fine. The screw has a length above the distance between the inside faces of the projections of the measuring rods and is fastened in one of its extremes in point 6.13 of the perforation body, and has the other extreme free and with a nurling in order that the surgeon can turn it to move away or bring the perforation body toward the projection.

Of the left projection, in the position that corresponds to the orifice with threading of the right projection, there is an orifice of the same dimensions through which a smooth rod 6.8 passes with a length below the opposite screw.

Toward the distal extreme of the projections, at the level where the distal orifice of the perforation body is located, in the two rods there are square slits of transveral section where the "basic instrument" will fit in. Near the distal extreme of the projections, these have a perforation through which a "scale gauge" (6.10) can be introduced that has the same length as that of the screw fixed to the perforation body. In the distal transversal face of the projections there are three prisoner screws 6.11, two to secure the basic body and one for the graduated ruler.

In the center of the upper surface (tibia) or side surface (femur), of this part "E" of the proximal body, near the distal edge, a perforation is provided 6.12 through which a strong prod will be passed, which during surgery goes through the skin and the first cortical of the patient's bone, in order to secure it to the perforated guide and avoid movements.

### Perforation Body

Separated from the proximal body, but secured to the projections of the measuring rods by means of the screw that includes said rods, is perforation body 6.13 which has a height above that of the guide (approximately 3 times) and has a rectangle paralelepiped configuration. In its distal face it has a center mark 6.14 in the form of a line in vertical direction. In the upper face and crossing it through the lower face it has two perforation orifices 6.15 with the same diameter and distance between them as the orifices of section D of the perforated guide, located in the center of the distance between centers of perforations in the center of the perforation body. Through those orifices enter the guides of the bolts and through these guides enter the drills.

### Basic Instrument

Is a rectangular section small gauge with level marks 7.1 that correspond to the levels of the vertical component of the guides and nails fastener. When the mark of the level coincides with the lower edge of the projections of the measuring arms, guide 7.2 inserts in a perforation located near to the inferior extreme of the basic instrument, will correspond to the most distal orifice of the distal orifices if the level is the same as the level of the vertical component where the perforated guide is lodged. In this position, it will be possible to make a scale drill 7.3 pass through guide 7.2 of the basic instrument which will reach as far as the surface of the nail implanted in the patient's bone, giving the distance there is from the basic instrument to the nail.

### Drills Guide and Bolts Guide

Bolt guides are metal tubes where the drill guides enter. In turn, these enter the orifices of the perforation body and have a sufficient length in order to reach the surface of the patient's bone. These drill guides are secured to the perforation body by two prisoner nails each.

The drill guides are the same as the bolt guides, but with an inferior diameter in order to lodge in these. Because of its perforation it enters a long drill. The system includes a set of four guides for bolts and four guides for drills, two for the orifices of the perforation body and two for the orifices of section D of the perforated guide.

### Description of the Surgical Technique followed in Using the System

1) The bone is opened at the proximal level in order to reach the marrow channel. Through this area the marrow drilling system is introduced in order to widen the channel until it has the sufficient diameter in order to permit the nail to enter the bone, by turning with the hand.
2) This technique is carried out by opening or not opening the point of the fracture.
3) The nail is introduced in the marrow channel passing it complete until the bumper of the horizontal part of the fastener hits the higher trochanter of the bone. The nail must not reach out beyond the bumper, nor must the bumper be introduced in the bone.
4) The perforated guide is placed at the level of the vertical component that can remain closer to the patient's skin.
5) The mark that corresponds to that of the number of the nail is placed in the distal edge of the "fastener".
6) The bolts are passed in the orifices of the sheet or soleplate above the perforated guide, through the corresponding orifices of the perforated guide and through the orifices of the sheet or inferior soleplate, which will leave the perforated guide perfectly placed and secured, and its distal part or section E mark the place of the distal orifices.
   **NOTE. With this placement there must be perfect correspondence between the orifices of the perforated guide and the orifices of the nail, but taking into account that, as mentioned before, the nails bend, one must use the "basic instrument" for measuring and then move the "perforation body" until it is placed in perfect correspondence with the orifices of the nail.**
7) The prod is introduced in the more distal orifice of the proximal body of the perforated guide until it passes the first cortical of the bone, which will leave the guide secured.
8) The basic instrument is placed on the corresponding measuring arm, according to whether it is the right or left bone.
9) A drill is introduced through the drills guide of the basic instrument that corresponds to the level selected in the fastener. When it reaches the skin an incision is made slightly above the diameter of the drill and is taken until the cortical of the bone is perforated and the drill touches the nail.
10) As this drill has scale marking, in the location where the drill juts out we have the distance indicating which will be the distance that exists between the tip of the drill, which is touching the nail, up to the location of the measurement.
11) The surgeon moves the small gauge from the perforation body toward the central mark, the distance provided by the drill with the scale marking.
12) **CAREFUL :** if the small gauge is not placed in the central mark, this means that the orifices of the nail and those of the bolt guide do not coincide. In this case, the surgeon turns the nail from the measuring arm the exact distance that makes the central mark of the perforation body coincide with the measure marked in the small gauge.
13) After the above maneuver, the bolt guides are introduced in the orifices of the perforation body and inside these the drill guides.
14) A drill is passed in one of the drill guides until it touches the skin and an incision is made, and the guides are passed until touching the bone. The cortical is perforated with the drill, then it passes through the orifice of the nail and then it perforates the second cortical of the bone. This drill is left in this position and the same operation is made in the other drill guide. The drills are left placed.
15) The bolt drill guides are placed in the orifices of the proximal body of the perforated guide (in their section D) which correspond to the proximal orifices of the nail and the same steps are performed as in the distal orifices.
16) The drill and drill guide are removed and a bolt with an adequate length is passed through the bolt guide screwed to the first cortical in each one of the orifices.
17) Drains are left and the wounds are closed.

## Claims

1. System for treating fractures and other injuries of the tibia, femur and humerus of the type that do not require an X-ray nor amplifiers, the system including: a) a securing body called vertical component (3.1, 3.2, 3.3, 3.6) with a rectangle parallelepiped configuration that in its two upper two thirds contains a series of longitudinal horizontal parallel cavities of square transveral section, which cross from its left face to its right face at different heights, and in its lower part, near its extreme and centered, it has an additional cavity from end to end which also in its inside has a tube (3.7) with a length that juts out, over the right face of the vertical component approximately 3% of its length and over the left face of the vertical component approximately one half of said length ; b) a rod (5.2) called horizontal component, which, lodged in the tube of the securing body, in the extreme that juts out in the left face of the securing body it has a cylindrical handle and in the extreme of the extreme that juts out in the right face of said securing body, it has an outside threading ; c) a square transversal section bar called perforated guide (6.1, 6.5) which, when lodged and secured in any of the cavities of the superior two thirds of the securing body has an extreme jutting out of the left face and the other extreme jutting out of its right face where it has two arms called measuring arms, where a perforation body is secured with a rectangle paralelepiped configuration with a pair of orifices that cross it vertically ; d) a bar called basic instrument (7.1) with scale marking at intervals that correspond to the levels of the parallel cavities of the securing body and secured in one of the arms of the perforated guide, moved toward its distal extreme, in a slot ; e) a set of metal tubes called bolt and drill guides (8.1, 8.2) in order to be placed in the orifices of the perforation body, with an outside diameter slightly less than the pair of orifices of the perforation body, the first and the second with an outside diameter slightly less than the inside diameter of the first, the second ; f) a set of nails (1.2), preferably cylindrical, with a pair of orifices near the distal extreme, and a pair of orifices closer toward its proximal extreme that has a longitudinal female threading that runs from its proximal face toward the inside of the nail and through which it is threaded to the threading of the horizontal component; and g) a set of bolts (2.3), most of them are cylindrical, with an extreme in the form of a truncated cone with the base toward the outside, the troncoconic extreme is threaded in order to introduce the cylindrical part in the nail implanted in the marrow channel toward the second cortical of the bone and that the threading be screwed in the first cortical of the bone, which will leave the nail secured.

2. System for the treatment of fractures and other injuries of the tibia, femur and humerus, such as claimed in the preceding claim, wherein the series of longitudinal horizontal parallel cavities of square transversal section of the securing body are four, and the three superior ones are formed by rectangular transversal section profiles, where the profiles that form the front and back faces are mounted on the side extremes of the inferior profile that corresponds to each cavity.

3. System for treating fractures and other injuries of the tibia, femur and humerus, such as claimed in the preceding claim, wherein the lower and upper profiles that form the cavities have a series of equidistant orifices in order to pass a bolt through them that, for the purpose of securing the perforated guide in the cavity.

4. System for the treatment of fractures and other injuries of the tibia, femur and humerus, such as claimed in any of the preceding claims, where also the lowest cavity of the securing body is formed by projections from the base of the securing body and only the upper face is a rectangular profile as those of the upper cavities.

5. System for the treatment of fractures and other injuries of the tibia, femur and humerus, such as claimed in any of the preceding claims, wherein the tube of the securing body has an increase in its outside diameter in its distal extreme that corresponds to the right face of the securing body.

6. System for the treatment of fractures and other injuries of the tibia, femur and humerus, such as claimed in any of the preceding claims, wherein the perforated guide has five well differentiated sections throughout its length, the most proximal part is a smooth segment and without orifices ; the second section, toward the proximal, is smooth and has a series of perforations equidistant of its upper face toward its lower face ; the third section, in addition to also having a series of holes as those of the second section, has engraved transveral lines equidistant between each hole with a number that corresponds to the nail number located there or that will be implanted ; the fourth section, similar to the first, with only a pair of perforations, with a separation between them equal to the separation of the proximal orifices of the nails, with the first orifice at such a distance from the distal extreme of the third segment that when the system is used there is total correspondence with the proximal orifices of the nails ; finally, the most distal segment of the guide has in its extremes the arms called measuring arms, between which the perforating body is secured, this distal extreme has a "P" mark and an orifice through which a prod will be inserted in order to secure the guide to the first cortical of the bone to be treated; wherein each measuring arm has an orifice loaded toward the distal extreme through which a scale gauge will be passed, its distance toward the most distal extreme of the arm is such that this gauge, when moved in its orifice, shifts over the distal face of the perforation body.

7. System for the treatment of fractures and other injures of the tibia, femur and humerus, such as claimed in any of the preceding claims, wherein the perforation body has in its distal face a center mark in the form of a vertical line ; said perforation body is secured to the measuring arms of the perforated guide by means of a scale-marked screw that crosses one of the measuring arms, said scale-marked screw juts out from the corresponding measuring arm to the front face of the perforation body and permits said perforation body to shift in the horizontal plane, on the face opposite to where the scale-marked screw is secured a smooth rod that glides across the opposite measuring arm.

8. System for the treatment of fractures and other injures of the tibia, femur and humerus, such as claimed in any of the preceding claims, wherein the basic instrument has, close to its inferior extreme, a perforation that contains a drill guide with scale marking that will permit a scale-marked drill to pass through it ; the distance from the inferior extreme to the aforementioned cavity is such that when they coincide in the level mark, the level must also coincide with the level of the cavity of the securing body where the guide is secured, with the inferior edge of the measuring arm where the basic instrument is secured, the nail implanted will coincide with the scale-marked drill guide.

9. System for the treatment of fractures and other injuries of the tibia, femur and humerus, such as claimed in any of the preceding claims, wherein regardless of the number nor of the type of nails, the distance between the first and second proximal orifices of the nail is the same as between the first and second orifices of the fourth section of the perforated guide and the distance between the first and second orifice of the distal orifices of the nail is the same as between the perforation orifices of the perforation body and the distance from the second proximal orifice of the nail and the first distal is the same as the distance from the second orifice, from proximal to distal, of the fourth section of the perforated guide and the proximal orifice of the perforation body.

10. System for the treatment of fractures and other injuries of the tibia, femur and humerus, such as claimed in any of the preceding claims, wherein the distance between the first and second distal orifice and the first and second proximal orifice of the nail is constant for any number and type of nail and will be the same as the distance between first and second orifice of the fourth section of the perforated guide and the distance between the perforation orifices of the perforation body and is the same as the distance between the perforation orifices of the perforation body and between the first and second orifice of the fourth section of the perforated guide.

11. System for the treatment of fractures and other injuries of the tibia, femur and humerus, such as claimed in any of the preceding claims, wherein the distance from the second distal orifice of the nail to the distal extreme of the nail is a constant for any type and number of nail.

12. System for the treatment of fractures and other injuries of the tibia, femur and humerus, such as claimed in any of the preceding claims, wherein the distance between the second proximal orifice and the first distal orifice of the nail for femur and tibia is a constant for any number of nail and equal to the distance from second orifice, from proximal to distal, of the fourth section of the perforated guide and the proximal orifice of the perforation body.

13. System for the treatment of fractures and other injuries of the tibia, femur and humerus, such as claimed in any of the preceding claims, wherein the distance between the second proximal orifice and the first distal orifice of the nails for humerus is smaller than in the case of the femur and tibia, but constant for any number and with the same distance from the second orifice, from proximal to distal, of the fourth section of the perforated guide and the proximal orifice of the perforation body.

14. System for the treatment of fractures and other injuries of the tibia, femur and humerus, such as claimed in any of the preceding claims, wherein the number of the nail will be correspondent with the distance between the first proximal orifice and the proximal extreme of the nail and will be equivalent to the distance between the first orifice of the fourth section of the perforated guide and the transversal guides of the third section of the perforated guide.

## Patentansprüche

1. Vorrichtung zur Behandlung von Frakturen und anderen Verletzungen des Schienbeins, des Oberschenkelknochens und des Oberarmknochens, die weder eine Röntgenaufnahme noch Verstärker benötigen, wobei die Vorrichtung umfasst: a) ein senkrechtes Bauteil (3.1, 3.2, 3.3, 3.6) genanntes Befestigungselement, das die Form eines Quaders aufweist, welches in seinen zwei oberen Zweidritteln eine Anzahl länglicher horizontal, parallel verlaufender Hohlräume mit quadratischem Querschnitt aufweist, die sich auf verschiedenen Höhen von seiner linken Seite zu seiner rechten Seite hin erstrecken und das in seinem unteren Teil im Endbereich einen zusätzlichen mittig vorgesehenen Hohlraum aufweist, der von dem einen zum anderen Ende reicht und in dem ein Rohr (3.7) untergebracht ist, dessen Länge derart gewählt ist, dass es über die rechte Seite des senkrechten Bauteils um etwa 3 % seiner Länge und über die linke Seite des senkrechten Bauteils um etwa die Hälfte seiner Länge hinausragt; b) eine in dem Rohr des Befestigungselements untergebrachtes horizontales Bauteil genannte Stange (5.2), die in dem aus der linken Seite des Befestigungselements herausragenden Endbereich einen zylindrischen Griff und in dem von der rechten Seite des Befestigungselements herausragenden Endbereich ein Aussengewinde aufweist; c) eine gelochte Führung (6.1, 6.5) genannte Stange mit einem quadratischen Querschnitt, die, wenn in einem beliebigen Hohlraum des oberen Zweidrittels des Befestigungselements untergebracht und befestigt, mit einem Ende aus der linken Seite herausragt, wobei das andere Ende aus der rechten Seite herausragt, wo sie zwei Messarme genannte Arme ausbildet, wobei ein Perforationselement an einem Quader mit zwei senkrecht durchgehenden Öffnungen befestigt ist; eine Grundgerät genannte Stange (7.1) mit einer Skala, wobei die Markierungen den verschiedenen Höhen der parallelen Hohlräume des Befestigungselements entsprechen, die in einem der Arme der gelochten Führung in einem Schlitz am distalen Ende befestigt ist; e) einen Satz Bolzen- und Bohrerführungen (8.1, 8.2) genannter Metallrohre zum Einsetzen in die Löcher des gelochten Elements, mit einem Aussendurchmesser, der etwas geringer ist als der der zwei Öffnungen im gelochten Element, wobei das erste und das zweite einen Aussendurchmesser aufweisen, der etwas geringer ist als der Innendurchmesser der ersten bzw. der zweiten; f) einen Satz Nägel (1.2), die vorzugsweise zylindrisch sind, mit zwei Öffnungen in der Nähe des distalen Endbereichs und zwei dem proximalen Ende näher gelegenen Öffnungen und mit einem längsgerichteten Innengewinde, das sich von der proximalen Seite des Nagels in das Innere desselben erstreckt und über welches es mit dem Gewinde des horizontalen Bauteils in Eingriff steht; und g) einem Satz Bolzen (2.3), von denen die meisten zylindrisch sind und die ein kegelstumpfförmiges Ende aufweisen, dessen Grundfläche nach Aussen gerichtet ist und das ein Gewinde aufweist, um den zylindrischen Teil in den in den Markkanal implantierten Nagel in Richtung auf die zweite Knochenrinde einzuführen und das Gewinde in die erste Knochenrinde eingeschraubt wird, wodurch der Nagel befestigt wird.

2. Vorrichtung zur Behandlung von Frakturen und anderen Verletzungen des Schienbeins, des Oberschenkelknochens und des Oberarmknochens nach dem vorstehenden Anspruch, wobei die Anzahl der länglichen horizontalen, parallel verlaufenden Hohlräume mit quadratischem Querschnitt des Befestigungskörpers vier beträgt und dass die drei oberen durch Profile mit rechteckigem Querschnitt gebildet sind, wobei die die Vorder- und Rückseite bildenden Profile auf die seitlichen Enden des dem jeweiligen Hohlraum entsprechenden unteren Profils montiert sind.

3. Vorrichtung zur Behandlung von Frakturen und anderen Verletzungen des Schienbeins, des Oberschenkelknochens und des Oberarmknochens nach dem vorstehenden Anspruch, wobei die die Hohlräume bildenden unteren und oberen Profile eine Reihe gleich beabstandeter Öffnungen aufweisen, durch welche, zum Befestigen der gelochten Führung in dem Hohlraum, ein Bolzen durchführbar ist.

4. Vorrichtung zur Behandlung von Frakturen und anderen Verletzungen des Schienbeins, des Oberschenkelknochens und des Oberarmknochens nach einem der vorstehenden Ansprüche, wobei auch der unterste Hohlraum des Befestigungskörpers durch Vorsprünge an der Basis des Befestigungskörpers gebildet wird, und nur die Oberseite wie die der oberen Hohlräume ein rechteckiges Profil ist.

5. Vorrichtung zur Behandlung von Frakturen und anderen Verletzungen des Schienbeins, des Oberschenkelknochens und des Oberarmknochens nach einem der vorstehenden Ansprüche, wobei das Rohr des Befestigungskörpers in seinem distalen Endbereich einen vergrösserten Aussendurchmesser aufweist, welcher der rechten Seite des Befestigungskörpers entspricht.

6. Vorrichtung zur Behandlung von Frakturen und anderen Verletzungen des Schienbeins, des Oberschenkelknochens und des Oberarmknochens nach einem der vorstehenden Ansprüche, wobei die gelochte Führung über ihre gesamte Länge fünf gut von einander zu unterscheidende Abschnitte aufweist, wobei der am nächsten gelegene Teil ein glattes Segment ohne Löcher ist, der zweite proximal gelegene Abschnitt glatt ist und eine Reihe Löcher aufweist, die im gleichen Abstand zu ihrer Oberseite und zu ihrer Unterseite angeordnet sind, der dritte Abschnitt zusätzlich zu den Löchern, die wie in dem zweiten Abschnitt angebracht sind, noch eingeritzte Querlinien aufweist, die mittig zwischen jedem Loch angebracht sind und deren Zahl der Anzahl an dort befindlichen oder noch zu implantierenden Nägeln entspricht, der vierte Abschnitt, ähnlich wie der erste nur zwei Löcher aufweist, die in der gleichen Weise wie die proximalen Öffnungen der Nägel voneinander getrennt sind, wobei die erste Öffnung derart von dem distalen Ende des dritten Segments beabstandet ist, dass, wenn die Vorrichtung in Gebrauch ist, eine völlige Übereinstimmung mit den proximalen Öffnungen der Nägel besteht und schliesslich das weitest entfernte Segment der Führung endseitig die Messarme genannten Arme aufweist, zwischen denen das perforierende Element befestigt ist, wobei dieses distale Ende eine "P"-Markierung sowie eine Öffnung aufweist, durch welche eine Ahle zur Befestigung der Führung an die erste zu behandelnde Knochenrinde eingeführt wird, und dass jeder Messarm eine Öffnung aufweist, die zum distalen Ende hin belastet ist und durch welche eine mit einer Skala versehene Messvorrichtung geführt wird, wobei deren Abstand zum weitest entfernten Ende des Arms derart ist, dass diese Messvorrichtung, wenn sie in ihrer Öffnung bewegt wird, über die distale Seite des Perforationselements verschoben wird.

7. Vorrichtung zur Behandlung von Frakturen und anderen Verletzungen des Schienbeins, des Oberschenkelknochens und des Oberarmknochens nach einem der vorstehenden Ansprüche, wobei das Perforationselement auf seiner distalen Seite eine mittig angebrachte Markierung in Form einer senkrechten Linie aufweist, wobei das Perforationselement mittels einer durch einen der Messarme der gelochten Führung hindurchragenden, mit einer Skala versehenen Schraube an die Messarme befestigt ist, wobei die mit einer Skala versehene Schraube aus dem entsprechenden Messarm in Richtung auf die Vorderseite des Perforationselements herausragt und dadurch das Perforationselement in der horizontalen Ebene verschiebt, wobei auf der der mit einer Skala versehenen Schraube gegenüberliegenden Seite eine glatte Stange befestigt ist, welche quer über den gegenüberliegenden Messarm verschiebbar gelagert ist.

8. Vorrichtung zur Behandlung von Frakturen und anderen Verletzungen des Schienbeins, des Oberschenkelknochens und des Oberarmknochens nach einem der vorstehenden Ansprüche, wobei das Grundgerät in der Nähe seines unteren Endes eine Perforierung aufweist, in der eine Bohrerführung mit einer Skala angebracht ist, durch welche ein mit einer Skala versehener Bohrer hindurchgeführt werden kann, wobei der Abstand zwischen dem unteren Ende und dem oben genannten Hohlraum derart gewählt ist, dass bei Übereinstimmung der Höhenmarkierungen, die Höhe auch mit der Höhe des Hohlraums im Befestigungselement, in dem die Führung befestigt ist, und mit der unteren Kante des Messarms, an der das Grundgerät befestigt ist, übereinstimmen muss, und der implantierte Nagel mit der mit einer Skala versehenen Bohrerführung übereinstimmt.

9. Vorrichtung zur Behandlung von Frakturen und anderen Verletzungen des Schienbeins, des Oberschenkelknochens und des Oberarmknochens nach einem der vorstehenden Ansprüche, wobei unabhängig von der Anzahl und der Art der Nägel, der Abstand zwischen der ersten und der zweiten proximalen Öffnung des Nagels der gleiche ist, wie der zwischen der ersten und der zweiten Öffnung des vierten Abschnitts der gelochten Führung und der Abstand zwischen der ersten und der zweiten Öffnung der distalen Öffnungen des Nagels der gleiche ist wie der Abstand zwischen den Perforierungen des Perforationselements und der Abstand zur zweiten proximalen Öffnung des Nagels und der ersten distalen der gleiche ist wie der Abstand zur zweiten Öffnung von proximal nach distal des vierten Abschnitts der gelochten Führung und zur proximalen Öffnung des Perforationselements.

10. Vorrichtung zur Behandlung von Frakturen und anderen Verletzungen des Schienbeins, des Oberschenkelknochens und des Oberarmknochens nach einem der vorstehenden Ansprüche, wobei der Abstand zwischen der ersten und der zweiten distalen Öffnung und der ersten und zweiten proximalen Öffnung des Nagels unabhängig von der Anzahl und der Art des Nagels konstant bleibt und der gleiche ist wie der Abstand zwischen der ersten und der zweiten Öffnung des vierten Abschnitts der gelochten Führung und der Abstand zwischen den Perforierungen des Perforationselements und der gleiche ist wie der Abstand zwischen den Perforierungen des Perforationselements und zwischen der ersten und der zweiten Öffnung des vierten Abschnitts der gelochten Führung.

11. Vorrichtung zur Behandlung von Frakturen und anderen Verletzungen des Schienbeins, des Oberschenkelknochens und des Oberarmknochens nach einem der vorstehenden Ansprüche, wobei der Abstand zwischen der zweiten distalen Öffnung des Nagels und dem distalen Ende des Nagels unabhängig von der Art und der Anzahl der Nägel konstant bleibt.

12. Vorrichtung zur Behandlung von Frakturen und anderen Verletzungen des Schienbeins, des Oberschenkelknochens und des Oberarmknochens nach einem der vorstehenden Ansprüche, wobei der Abstand zwischen der zweiten proximalen Öffnung und der ersten distalen Öffnung des Nagels für Oberschenkelknochen und Schienbein unabhängig von der Anzahl der Nägel konstant bleibt und dem Abstand zwischen der zweiten Öffnung, von proximal nach distal, des vierten Abschnitts der gelochten Führung und der proximalen Öffnung des Perforationselements entspricht.

13. Vorrichtung zur Behandlung von Frakturen und anderen Verletzungen des Schienbeins, des Oberschenkelknochens und des Oberarmknochens nach einem der vorstehenden Ansprüche, wobei der Abstand zwischen der zweiten proximalen Öffnung und der ersten distalen Öffnung der Nägel für den Oberarmknochen geringer ist, als beim Oberschenkelknochen und beim Schienbein, aber unabhängig von der Anzahl konstant bleibt und mit dem gleichen Abstand zur zweiten Öffnung, von proximal nach distal, des vierten Abschnitts der gelochten Führung und zur proximalen Öffnung des Perforationselements aufweist.

14. Vorrichtung zur Behandlung von Frakturen und anderen Verletzungen des Schienbeins, des Oberschenkelknochens und des Oberarmknochens nach einem der vorstehenden Ansprüche, wobei die Anzahl der Nägel entsprechend dem Abstand zwischen der ersten proximalen Öffnung und dem proximalen Ende des Nagels gewählt wird und dem Abstand zwischen der ersten Öffnung des vierten Abschnitts der gelochten Führung und den querverlaufenden Führungen des dritten Abschnitts der gelochten Führung entspricht.

## Revendications

1. Système destiné au traitement de fractures et autres blessures du tibia, du fémur et de l'humérus du type ne nécessitant pas de rayons X ni d'amplificateur et comportant : a) un élément de fixation appelé pièce constituante verticale (3.1, 3.2, 3.3, 3.6) conformée en forme de parallélépipède rectangle qui comporte, dans les deux tiers supérieurs, une série de cavités parallèles horizontales longitudinales de section transversale carrée qui s'étendent à différents niveaux depuis son côté gauche vers son côté droit et dans sa partie inférieure, près de son extrémité, une cavité supplémentaire centrée s'étendant d'une extrémité à l'autre et comportant en son sein un tube (3.7) d'une longueur telle qu'environ 3 % de sa longueur dépasse de la face droite de la pièce constituante verticale alors qu'environ une moitié de ladite longueur dépasse la face gauche de la pièce constituante verticale ; b) une barre (5.2) appelée pièce constituante horizontale qui, logée dans le tube de l'élément de fixation, comporte à son extrémité dépassant la face gauche de l'élément de fixation une poignée cylindrique et à son extrémité de l'extrémité qui dépasse de la face droite dudit élément de fixation un filetage mâle ; c) une barre de section transversale carrée appelée guide perforé (6.1, 6.5) qui, quand logé et fixé dans l'une quelconque des cavités des deux tiers supérieurs de l'élément de fixation, a une extrémité qui dépasse de la face gauche et l'autre extrémité qui dépasse de sa face droite où elle comporte deux bras appelés bras de mesure où est fixé un élément de perforation conformé en forme de parallélépipède rectangle muni d'une paire d'orifices le traversant verticalement ; d) une barre (7.1) graduée appelée instrument de base dont les graduations sont prévues à des intervalles correspondant aux niveaux de l'élément de fixation, ledit instrument de base étant fixé dans une fente dans l'un des bras du guide perforé à son extrémité distale ; e) un ensemble de tubes en métal appelé guides de perçage et guides-boulons (8.1, 8.2) destiné à être placé dans les orifices de l'élément de perforation, leur diamètre extérieur étant légèrement inférieur à celui de la paire d'orifices de l'élément de perforation, le premier et le second ayant un diamètre extérieur légèrement inférieur au diamètre intérieur du premier, du deuxième ; f) un ensemble de clous (1.2), de préférence cylindriques, avec une paire d'orifices situés près de l'extrémité distale et une paire d'orifices situés plus près de son extrémité proximale, ledit clou ayant un filetage femelle longitudinal s'étendant depuis sa face proximale vers l'intérieur du clou et par lequel il est mis en prise avec le filetage de la pièce constituante horizontale ; et g) un ensemble de boulons (2.3) dont la plupart sont cylindriques, une extrémité étant conformée en forme de cône tronqué dont la base est tournée vers l'extérieur, l'extrémité tronconique étant filetée pour introduire la partie cylindrique dans le clou implanté dans le canal médullaire en direction du second cortex osseux et de visser le filetage dans le premier cortex osseux, fixant ainsi le clou.

2. Système destiné au traitement de fractures et autres blessures du tibia, du fémur et de l'humérus selon la revendication précédente, **caractérisé en ce que** les cavités parallèles horizontales longitudinales sont au nombre de quatre et que les trois supérieures sont formées par des profilés de section transversale rectangulaire, les profilés formant les faces avant et arrière étant montés sur les extrémités latérales du profilé inférieur correspondant à chacune des cavités.

3. Système destiné au traitement de fractures et autres blessures du tibia, du fémur et de l'humérus selon la revendication précédente, **caractérisé en ce que** les profilés inférieurs et supérieurs formant les cavités ont une série d'orifices équidistants destinés à y passer un boulon afin de fixer le guide perforé dans la cavité.

4. Système destiné au traitement de fractures et autres blessures du tibia, du fémur et de l'humérus selon l'une quelconque des revendications précédentes, **caractérisé en ce que** la cavité inférieure de l'élément de fixation est formée par des saillies s'étendant à partir de la base de l'élément de fixation, seule la face supérieure étant, comme celle des cavités supérieures, un profilé rectangulaire.

5. Système destiné au traitement de fractures et autres blessures du tibia, du fémur et de l'humérus selon l'une quelconque des revendications précédentes, **caractérisé en ce que** le diamètre extérieur du tube de l'élément de fixation augmente à son extrémité distale, correspondant à la face droite de l'élément de fixation.

6. Système destiné au traitement de fractures et autres blessures du tibia, du fémur et de l'humérus selon l'une quelconque des revendications précédentes, **caractérisé en ce que** le guide perforé a cinq sections bien distinctes réparties sur sa longueur, la partie la plus proximale étant un segment lisse sans orifices ; la seconde section, en proximal, étant lisse et pourvue d'une série de perforations ménagées à égale distance entre la face supérieure et la face inférieure ; la troisième section, elle aussi pourvue, comme la deuxième section, d'une série de trous, ayant des lignes transversales équidistantes gravées entre chacun des trous dont le nombre correspond au nombre de clous qui s'y trouvent ou qui sont destinés à être implantés ; la quatrième section, similaire à la première, munie uniquement d'une paire de perforations, lesdites perforations étant séparées de la même manière que les orifices proximaux des clous, le premier orifice étant situé à une distance de l'extrémité distale du troisième segment telle que, quand le système est en service, il y a correspondance totale avec les orifices proximaux des clous ; le segment le plus distal du guide portant finalement à ses extrémités les bras appelés bras de mesure entre lesquels est fixé l'élément perforant, ladite extrémité distale étant pourvue d'une marque « P » et d'un orifice par lequel sera inséré un poinçon pour fixer le guide au premier cortex osseux à traiter ; que chaque bras de mesure a un orifice sollicité en direction de l'extrémité distale et par lequel on fera passer une jauge étalonnée, sa distance par rapport à l'extrémité la plus distale du bras étant telle que cette jauge, lorsqu'on la fait bouger dans son orifice, se déplace sur la face distale de l'élément de perforation.

7. Système destiné au traitement de fractures et autres blessures du tibia, du fémur et de l'humérus selon l'une quelconque des revendications précédentes, **caractérisé en ce que** l'élément de perforation comporte dans sa face distale une marque centrale en forme de ligne verticale ; ledit élément de perforation est fixé aux bras de mesure du guide perforé au moyen d'une vis graduée qui traverse l'un des bras de mesure, la vis graduée dépassant le bras de mesure correspondant en direction de la face frontale de l'élément de perforation et permettant le déplacement de l'élément de perforation dans le plan horizontal, une barre lisse coulissant en travers du bras de mesure opposé sur la face opposée à l'endroit où est fixée la vis graduée.

8. Système destiné au traitement de fractures et autres blessures du tibia, du fémur et de l'humérus selon l'une quelconque des revendications précédentes, **caractérisé en ce que** l'instrument de base comporte, près de son extrémité inférieure, une perforation contenant un guide de perçage étalonné permettant le passage d'un foret gradué, la distance entre l'extrémité inférieure et la cavité mentionnée ci-avant étant telle que, lorsque leurs graduations coïncident, le niveau doit aussi coïncider avec le niveau de la cavité de l'élément de fixation dans laquelle est fixé le guide, avec le bord inférieur du bras de mesure auquel est fixé l'instrument de base, le clou implanté coïncidant avec le guide de perçage étalonné.

9. Système destiné au traitement de fractures et autres blessures du tibia, du fémur et de l'humérus selon l'une quelconque des revendications précédentes, **caractérisé en ce que**, quel que soit le nombre ou le type de clous utilisés, la distance entre le premier et le deuxième orifice proximal du clou est égale à celle entre le premier et le deuxième orifice de la quatrième section du guide perforé et la distance entre le premier et le deuxième orifice des orifices distaux du clou est égale à celle entre les orifices de perforation de l'élément de perforation et la distance du second orifice proximal du clou et du premier orifice distal est égale à la distance du deuxième orifice, de proximal vers distal, de la quatrième section du guide perforé et de l'orifice proximal de l'élément de perforation.

10. Système destiné au traitement de fractures et autres blessures du tibia, du fémur et de l'humérus selon l'une quelconque des revendications précédentes, **caractérisé en ce que**, indépendamment du nombre et du type de clou utilisé, la distance entre le premier et le deuxième orifice distal et le premier et le deuxième orifice proximal du clou est constante et est égale à la distance entre le premier et le deuxième orifice de la quatrième section du guide perforé et la distance entre les orifices de perforation de l'élément de perforation est égale à la distance entre les orifices de perforation de l'élément de perforation et entre le premier et le deuxième orifice de la quatrième section du guide perforé.

11. Système destiné au traitement de fractures et autres blessures du tibia, du fémur et de l'humérus selon l'une quelconque des revendications précédentes, **caractérisé en ce que** la distance entre le deuxième orifice distal du clou et l'extrémité distale du clou est constante quel que soit le type et le nombre de clous utilisés.

12. Système destiné au traitement de fractures et autres blessures du tibia, du fémur et de l'humérus selon la revendication précédente, **caractérisé en ce que**, dans le cas du fémur et du tibia, la distance entre le deuxième orifice proximal et le premier orifice distal du clou est constante quel que soit le nombre de clous et est égale à la distance du deuxième orifice, de proximal vers distal, de la quatrième section du guide perforé et de l'orifice proximal de l'élément de perforation.

13. Système destiné au traitement de fractures et autres blessures du tibia, du fémur et de l'humérus selon l'une quelconque des revendications précédentes, **caractérisé en ce que**, dans le cas de l'humérus, la distance entre le deuxième orifice proximal et le premier orifice distal des clous est inférieure à celle dans le cas du fémur et du tibia, mais est constante quel que soit le nombre, la distance du deuxième orifice, de proximal vers distal, de la quatrième section du guide perforé et de l'orifice proximal de l'élément de perforation étant la même.

14. Système destiné au traitement de fractures et autres blessures du tibia, du fémur et de l'humérus selon l'une quelconque des revendications précédentes, **caractérisé en ce que** le nombre de clous correspond à la distance entre le premier orifice proximal et l'extrémité proximale du clou et équivaut à la distance entre le premier orifice de la quatrième section du guide perforé et les guides transversaux de la troisième section du guide perforé.
